# EUROPÄISCHE PATENTSCHRIFT

(11) **EP 2 308 822 B1**
(45) Veröffentlichungstag und Bekanntmachung des Hinweises auf die Patenterteilung: **31.08.2016**
(21) Anmeldenummer: 10009852.4
(22) Anmeldetag: 17.09.2010
(51) Int. Cl.: C07C 67/08, C07C 67/60, C07C 69/28

(54) **Verfahren zur Farbaufhellung von Polyolestern**
Method for brightening the colour of polyol esters
Procédé d'éclaircissement d'esters de polyols

(30) Priorität: 08.10.2009 DE 102009048773
(43) Veröffentlichungstag der Anmeldung: 13.04.2011
(73) Patentinhaber: OXEA GmbH, 46147 Oberhausen (DE)
(72) Erfinder: Frey, Guido, D., Dr., 64560 Riedstadt (DE); Kreickmann, Thorsten, Dr., 46149 Oberhausen (DE); Weber, Tonia, Dr., 64293 Darmstadt (DE); Strutz, Heinz, Dr., 47445 Moers (DE)

(56) Entgegenhaltungen:
- WO-A1-94/18153
- DD-A- 57 596
- DE-A1- 3 240 892
- DE-A1- 3 623 371

## Beschreibung

Die Erfindung betrifft ein Verfahren zur Farbaufhellung von Polyolestern aus linearen oder verzweigten aliphatischen Monocarbonsäuren mit 3 bis 20 Kohlenstoffatomen durch Behandlung des Polyolesters mit peroxidischen Verbindungen.

Ester mehrwertiger Alkohole, auch Polyolester genannt, finden in großem Umfang und in vielfältiger Weise Anwendung in der Technik, wie zum Beispiel als Weichmacher oder Schmiermittel. Durch die Auswahl geeigneter Ausgangsprodukte lassen sich die physikalischen Stoffeigenschaften, wie zum Beispiel Siedepunkt oder Viskosität gezielt einstellen, und den chemischen Eigenschaften, wie der Hydrolyseresistenz oder der Stabilität gegenüber einem oxidativen Abbau Rechnung tragen. Auch auf die Lösung konkreter anwendungstechnischer Probleme können Polyolester gezielt zugeschnitten werden. Ausführliche Übersichten über den Einsatz von Polyolestern finden sich zum Beispiel in Ullmann's Encyclopedia of Industrial Chemistry, 5. Auflage, 1985, VCH Verlagsgesellschaft, Vol. A1, Seiten 305-319; 1990, Vol. A15, Seiten 438-440 oder in Kirk Othmer, Encyclopedia of Chemical Technology, 3. Auflage, John Wiley & Sons, 1978, Vol. 1, Seiten 778-787; 1981, Vol. 14, Seiten 496-498.

Die Verwendung von Polyolestern als Schmierstoffe besitzt eine große technische Bedeutung und sie werden besonders für solche Anwendungsgebiete eingesetzt, in denen Schmierstoffe auf Mineralölbasis die gesetzten Anforderungen nur unvollständig erfüllen. Polyolester werden insbesondere als Turbinenmotoren- und Instrumentenöle benutzt. Polyolester für Schmiermittelanwendungen basieren häufig auf 1,3-Propandiol, 1,3-Butandiol, 1,4-Butandiol, 1,2-Hexandiol, 1,6-Hexandiol, Neopentylglykol, Trimethylolpropan, Pentaerythrit, 2,2,4-Trimethylpentan-1,3-diol, Glycerin oder 3(4),8(9)-Di-hydroxymethyltricyclo[5.2.1.0^{2.6}]decan, auch als TCD-Alkohol DM bezeichnet, als Alkoholkomponente.

Auch als Weichmacher werden Polyolester in erheblichem Umfang verwendet. Weichmacher finden in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihre Löse- und Quellvermögen in physikalische Wechselwirkung. Hierdurch bildet sich ein homogenes System aus, dessen thermoplastischer Bereich gegenüber den ursprünglichen Polymeren zu niederen Temperaturen verschoben ist, u. a. mit dem Ergebnis, dass seine mechanischen Eigenschaften optimiert, z. B. Formveränderungsvermögen, Elastizität, Festigkeit erhöht werden und die Härte verringert wird.

Um Weichmachern möglichst weite Anwendungsgebiete zu eröffnen, müssen sie eine Reihe von Kriterien erfüllen. Im Idealfall sollten sie geruchlos, farblos, licht-, kälte- und wärmebeständig sein. Überdies erwartet man, dass sie unempfindlich gegenüber Wasser, schwer brennbar und wenig flüchtig sind und die Gesundheit nicht schädigen. Weiterhin soll die Herstellung der Weichmacher einfach sein und, um ökologischen Anforderungen zu genügen, unter Vermeidung von Abfallstoffen, wie nicht weiterverwertbaren Nebenprodukten und Schadstoffe enthaltenden Abwässern, erfolgen. Eine spezielle Klasse von Polyolestern (sie werden kurz als G-Ester bezeichnet) enthält als Alkoholkomponente Diole bzw. Etherdiole, beispielsweise Ethylenglykol, Diethylenglykol, Triethylenglykol, Tetraethylenglykol, 1,2-Propylenglykol oder höhere Propylenglykole. Ihre Herstellung kann auf unterschiedlichen Wegen erfolgen. Neben der Umsetzung von Alkohol und Säure gegebenenfalls in Gegenwart saurer Katalysatoren werden in der Praxis weitere Prozesse zur Gewinnung von G-Estern angewandt, u. a. die Reaktion von Diol mit Säurehalogenid, die Umesterung eines Carbonsäureesters mit einem Diol und die Addition von Ethylenoxid an Carbonsäuren (Ethoxylierung). In der industriellen Fertigung haben sich lediglich die unmittelbare Umsetzung von Diol und Carbonsäure und die Ethoxylierung von Carbonsäuren als Produktionsverfahren durchgesetzt, wobei der Veresterung von Diol und Säure zumeist der Vorzug gegeben wird. Denn dieses Verfahren kann ohne besonderen Aufwand in konventionellen chemischen Apparaten durchgeführt werden und es liefert chemisch einheitliche Produkte. Demgegenüber erfordert die Ethoxylierung umfangreiche und kostenintensive technische Mittel. Ethylenoxid ist eine sehr reaktive chemische Substanz. Es kann explosionsartig polymerisieren und bildet mit Luft in sehr weiten Mischungsbereichen explosive Gemische. Ethylenoxid reizt die Augen und Atemwege, führt zu Verätzungen, zu Leber- und Nierenschäden und ist karzinogen. Seine Handhabung erfordert daher umfangreiche Sicherheitsmaßnahmen. Überdies ist auf peinliche Sauberkeit von Lagervorrichtungen und Reaktionsapparaten zu achten, um die Bildung unerwünschter Verunreinigungen durch Nebenreaktionen des Ethylenoxids mit Fremdstoffen auszuschließen. Schließlich ist die Reaktion mit Ethylenoxid nicht sehr selektiv, denn sie führt zu Gemischen von Verbindungen unterschiedlicher Kettenlänge.

Die unmittelbare Veresterung von Alkoholen mit Carbonsäuren gehört zu den Grundoperationen der organischen Chemie. Um die Reaktionsgeschwindigkeit zu erhöhen, führt man die Umsetzung üblicherweise in Gegenwart von Katalysatoren durch. Der Einsatz eines der Reaktanten im Überschuss und/oder die Abtrennung des im Verlauf der Reaktion gebildeten Wassers stellt sicher, dass das Gleichgewicht entsprechend dem Massenwirkungsgesetz zur Seite des Reaktionsproduktes, also des Esters, verschoben wird, d. h. hohe Ausbeuten erzielt werden.

Umfassende Angaben zur Herstellung von Estern mehrwertiger Alkohole, darunter auch Estern aus Ethylenglykolen und Fettsäuren und zu den Eigenschaften ausgewählter Vertreter dieser Verbindungsklassen finden sich in Goldsmith, Polyhydric Alcohol Esters of Fatty Acids, Chem. Rev. 33, 257 ff. (1943). Beispielsweise erfolgt die Herstellung von Estern des Diethylenglykols, des Triethylenglykols und von Polyethylenglykolen über Reaktionszeiten von 2,5 bis 8 Stunden bei Temperaturen von 130 bis 230°C. Zur Entfernung des Reaktionswassers verwendet man Kohlendioxid. Als geeignete Katalysatoren für die Veresterung mehrwertiger Alkohole werden anorganische Säuren, saure Salze, organische Sulfonsäuren, Acetylchlorid, Metalle oder amphotere Metalloxide genannt. Die Entfernung des Reaktionswassers erfolgt mit Hilfe eines Schleppmittels, beispielsweise Toluol oder Xylol oder durch Einleiten inerter Gase wie Kohlendioxid oder Stickstoff.

Auf die Gewinnung und die Eigenschaften von Fettsäureestern der Polyethylenglykole geht Johnson (Edit.), Fatty Acids in Industry (1989) Kap. 9, Polyoxyethylene Esters of Fatty Acid ein und gibt eine Reihe präparativer Hinweise. Höhere Diesterkonzentrationen erzielt man durch die Erhöhung des molaren Verhältnisses von Carbonsäure zu Glykol. Geeignete Maßnahmen zur Entfernung des Reaktionswassers sind die Azeotropdestillation in Gegenwart eines mit Wasser nicht mischbaren Lösungsmittels, das Erhitzen unter Durchleiten eines Inertgases oder die Durchführung der Reaktion unter Vakuum in Gegenwart eines Trockenmittels. Verzichtet man auf den Zusatz von Katalysatoren, so sind längere Reaktionszeiten und höhere Reaktionstemperaturen erforderlich. Beide Reaktionsbedingungen können durch den Einsatz von Katalysatoren gemildert werden. Neben Schwefelsäure sind organische Säuren wie p-Toluolsulfonsäure sowie Kationenaustauscher vom Polystyroltyp die bevorzugten Katalysatoren. Auch die Verwendung von Metallpulvern, wie Zinn oder Eisen, wird beschrieben. Nach der Lehre aus US 2,628,249 lassen sich Farbprobleme bei der Katalyse mit Schwefelsäure oder Sulfonsäuren mildern, wenn in Gegenwart von Aktivkohle gearbeitet wird.

Eine Arbeitsweise, bei der Ester des Diethylen- und Triethylenglykols und der Caprylsäure ohne Katalysatorzusatz hergestellt werden, ist aus US 2,469,446 bekannt. Die Veresterungstemperatur liegt im Bereich von 270 bis 275°C und das Reaktionswasser wird mittels eines Kohlendioxidstromes ausgetrieben.

Bei der Reaktionsführung, bei der man auf die Zugabe eines Katalysators verzichtet, arbeitet man im Allgemeinen mit einem molaren Überschuss an der jeweiligen Carbonsäure, die aufgrund ihrer Acidität auch als Katalysator wirkt.

Für die Abtrennung des bei der Esterbildung aus dem Polyol und der Carbonsäuren gebildeten Reaktionswassers sind verschiedene Verfahren bekannt. Beispielsweise wird das gebildete Reaktionswasser zusammen mit der überschüssigen Carbonsäure aus dem Reaktionsgefäß abdestilliert und in einen nachgeschalteten Phasentrenner geleitet, in dem sich Carbonsäure und Wasser je nach ihren Löslichkeitseigenschaften auftrennen. Gegebenenfalls bildet die eingesetzte Carbonsäure mit Wasser unter den Reaktionsbedingungen auch ein Azeotrop und vermag als Schleppmittel das Reaktionswasser zu entfernen. Anwendung findet auch die Azeotropdestillation in Gegenwart eines zugesetzten mit Wasser nicht mischbaren Lösemittels, das Erhitzen des Reaktionsgemisches unter Durchleiten eines inerten Gases, die Umsetzung der Ausgangsstoffe Polyol und Carbonsäure unter Vakuum oder in Gegenwart eines Trocknungsmittels. Insbesondere die Wasserentfernung durch azeotrope Destillation hat sich für die Einstellung des Gleichgewichts bei der Herstellung von Polyolestern bewährt. Nach der aus DE 199 40 991 A1 bekannten Arbeitsweise wird das mit Wasser nicht mischbare Lösemittel, das als Schleppmittel wirkt und das einen Siedepunkt von weniger als 112°C aufzuweisen hat, dem Reaktionsansatz erst bei Erreichen einer Temperatur von mindestens 140°C zugesetzt.

Im technischen Prozess wird das abgetrennte Gemisch aus Wasser und Carbonsäure in einem Phasentrenner in die organische und wässrige Phase geschieden, die wässrige Phase ausgeschleust und die Carbonsäure wieder in die Veresterungsreaktion zurückgeführt. Für die Aufarbeitung des Rohesters schlägt beispielsweise US 5,324,853 A1 vor, überschüssige Carbonsäure mittels Durchleiten von Stickstoff oder Wasserdampf zu entfernen, ein Adsorbens zuzusetzen, mit einer Base restliche organische Säure zu neutralisieren und angefallene Feststoffe abzufiltrieren. Die im Filtrat vorhandenen Restmengen an Säure werden mit dem Durchleiten von Wasserdampf oder Stickstoff bei gleichzeitigem Anlegen eines Unterdrucks entfernt und wieder in die Veresterungsreaktion zurückgeführt. Bei der Vakuumbehandlung angefallene Feststoffe werden in einer abschließenden Feinfiltration entfernt. Das zugesetzte Adsorbens, beispielsweise Aktivkohle, hat unter anderem die Aufgabe, die Farbe des Polyolesters zu verbessern.

Nach der aus US 2,469,446 A1 bekannten Arbeitsweise wird der nach Abtrennung des Reaktionswassers und überschüssiger, nicht umgesetzter Ausgangsstoffe, beispielsweise Carbonsäure, angefallene Rohester zunächst mit einem alkalischen Reagenz, zum Beispiel mit einer wässrigen Soda- oder Natriumhydroxidlösung behandelt, um letzte Reste acider Bestandteile zu entfernen. Nach Wasserwäsche, Behandlung mit Bleicherde und Aktivkohle kann zur Entfernung letzter Spuren von geruchsgebenden Stoffen Vakuum bei erhöhter Temperatur angelegt werden. Gegebenenfalls ist die Behandlung mit Bleichmitteln und Aktivkohle mehrmals zu wiederholen, um Polyolester mit zufriedenstellenden Farbeigenschaften zu produzieren. Maßnahmen zur Farbverbesserung von rohen Estern, wie die Oxidation, beispielsweise mit Wasserstoffperoxid oder Ozon, sowie die Adsorption von Aktivkohle sind aus dem allgemeinen Stand der Technik, beispielsweise aus H. Suter, Phthalsäureanhydrid und seine Verwendung, Dr. Dietrich Steinkopf Verlag, Darmstadt 1972, bekannt. Zur Farbverbesserung von Esterverbindungen auf Basis von Polyolen schlägt WO 94/18153 A1 eine nachträgliche Behandlung mit einer wässrigen Wasserstoffperoxidlösung vor. Das Einwirken von Ozon zur Farbaufhellung wird beispielsweise in DE 27 29 627 A1 behandelt.

DD 57 596 A offenbart ein Verfahren zur Reinigung von aromatischen Dicarbonsäureestern, bei dem das rohe Esterprodukt unter Beibehaltung der Veresterungstemperatur zunächst mit Alkali behandelt wird und nach Abtrennung des überschüssigen Alkohols im Unterdruck mit einer wässrigen Wasserstoffperoxidlösung versetzt wird. Es folgt eine Wasserdampfbehandlung bei vermindertem Druck.

Wegen der eingangs beschriebenen Qualitätskriterien für Polyolester sind die Verfahrensschritte bei der Veresterungsstufe unter Entfernung des Reaktionswassers und bei der Aufarbeitung des Rohesters sehr wesentliche Prozessmerkmale, denn die Abstimmung dieser Verfahrensschritte beeinflusst in wesentlichem Maße die sensorischen und optischen Eigenschaften der Endprodukte. Insbesondere werden hohe Anforderungen an die Farbeigenschaften, wie geringe Farbzahl und hohe Farbstabilität, der Polyolester gestellt. Die Struktur der Ausgangsstoffe, der mehrwertigen Alkohole und der Säuren, ist dagegen für die mechanischen und thermischen Eigenschaften der mit den Polyolestern weichgestellten Kunststoffmassen maßgebend und beeinflusst die Hydrolyse- und Oxidationsstabilität von Schmiermitteln.

Die Behandlung mit einem Adsorbens, beispielsweise Aktivkohle, oberflächenreiche Polykieselsäuren, wie Silicagele (Kiesel-Xerogele), Kieselgel, Kieselgur, oberflächenreiche Aluminiumoxide und Aluminiumoxidhydrate, mineralische Materialien, wie Tone oder Carbonate, während der Aufarbeitung des rohen Polyolesters zur Farbverbesserung ist ein gängiges Verfahren, es erfordert jedoch zusätzliche Filtrationsschritte, die bei einem technisch durchgeführten Verfahren einen erheblichen Aufwand bedeuten. Ebenfalls bleibt wertvolles Produkt in der Filtereinrichtung und auf dem Adsorbens haften, so dass bei einem zusätzlichen Filtrationsschritt wertvolles Produkte verloren geht.

Auch die Behandlung mit Wasserstoffperoxid zur Farbverbesserung kann sich als problematisch erweisen, da es zur Bildung organischer Peroxide während der Behandlung der Polyolester kommen kann. Spuren von Peroxiden mindern die Esterqualität und die anwendungstechnischen Eigenschaften der weichgestellten Kunststofferzeugnisse sowie der auf Basis von Polyolestern produzierten Schmiermittel. Auch beeinträchtigen Peroxidspuren das Lagerverhalten der Polyolester und man beobachtet während der Lagerung trotz Ausschluss von Oxidationsmitteln wie Luft eine Zunahme der Peroxidzahl. Zur Verminderung der Peroxidzahl schlägt der Stand der Technik eine zusätzliche Behandlung mit einem Reduktionsmittel vor. Dieses Verfahren vermag zwar die Peroxidzahl zu reduzieren, solches Vorgehen bedeutet aber einen zusätzlichen Arbeitsschritt, bei dem das Reduktionsmittel bereitgestellt und nach seinem Verbrauch wieder abgetrennt werden muss.

Es wurde nun gefunden, dass man bei der Behandlung des rohen Polyolesters mit peroxidischen Verbindungen zu hellfarbigen Produkten ohne Einsatz von Adsorbentien gelangen kann, wenn man eine Behandlung mit peroxidischen Verbindungen und unmittelbar anschließend ohne weitere Zwischenschritte eine Behandlung mit Wasserdampf durchführt und abschließend den Polyolester trocknet, wobei die Bedingungen während der Behandlungen, wie anzuwendende Temperatur, Dauer oder anzulegender Druck auf den jeweiligen Polyolester zugeschnitten werden.

Überraschenderweise wird bei dieser Arbeitsweise ein hellfarbiger Polyolester erhalten, der eine äußerst geringe Peroxidzahl aufweist, die selbst über einen längeren Lagerungszeitraum stabil bleibt und nicht zunimmt.

Die Erfindung besteht daher in einem Verfahren zur Farbaufhellung von Polyolestern der allgemeinen Formel

H-(-O-[-CR¹R²-]ₘ-)ₒ-OH

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten, durch Umsetzung von Polyolen mit linearen oder verzweigten aliphatischen Monocarbonsäuren mit 3 bis 20 Kohlenstoffatomen im Überschuss und anschließende Aufarbeitung des Reaktionsgemisches ohne die Verwendung von Adsorbentien. Das Verfahren ist dadurch gekennzeichnet, dass nach Abtrennung unumgesetzter Ausgangsverbindungen das Umsetzungsprodukt mit peroxidischen Verbindungen, ausgewählt aus Wasserstoffperoxid, Alkali- oder Erdalkalimetallperboraten, Alkali- oder Erdalkalimetallpercarbonaten und Alkali- oder Erdalkalimetallperoxophosphaten, mit einem Wirkgehalt von 0,03 bis 0,3 Gew.-%, bezogen auf die gesamte Mischung, behandelt wird, unmittelbar anschließend ohne weitere Zwischenschritte eine Wasserdampfbehandlung bei einer Temperatur von 170 bis 220°C und über einen Zeitraum von 0,5 bis 5 Stunden durchgeführt wird und der zurückgebliebene Polyolester getrocknet wird, mit der Maßgabe, dass die aliphatische Monocarbonsäure einen niedrigeren Siedepunkt als das eingesetzte Polyol aufweist.

Die neue Arbeitsweise zeichnet sich durch große Zuverlässigkeit nicht nur im Labor- und Versuchsbetrieb sondern vor allem auch in technischen Anlagen aus. Sie ist, auch kontinuierlich, leicht durchzuführen und liefert Polyolester mit hoher Reinheit. Die Behandlung des Rohesters mit peroxidischen Verbindungen mit unmittelbar anschließender Wasserdampfbehandlung und weiterer Trocknung führt zu ausgezeichneten Farbeigenschaften und bemerkenswerter Farbstabilität von Polyolestern, die zudem nur eine geringe Peroxidzahl aufweisen. Auch bleibt die Peroxidzahl über eine längere Lagerzeit stabil auf einem niedrigen Niveau.

Für die Behandlung des nach Abtrennung unumgesetzter Ausgangsverbindungen angefallenen Rohesters eignen sich als peroxidische Verbindungen Wasserstoffperoxid, Alkali- oder Erdalkalimetallperborate, Alkali- oder Erdalkalimetallpercarbonate oder Alkali- oder Erdalkalimetallperoxophoshate.

Besonders geeignet sind wässrige Wasserstoffperoxidlösungen, die auf einfache Weise destillativ abgetrennt werden können. Die Anwendung der salzartigen, peroxidischen Alkali- oder Erdalkalimetallverbindungen, entweder in fester Form oder als wässrige Lösung, ist nicht ausgeschlossen aber auf wenige Ausnahmefälle beschränkt, da sie und ihre Umsetzungsprodukte als Feststoffe vorliegen oder im Zuge der Aufarbeitung des Rohesters sich niederschlagen und durch einen zusätzlichen Filtrationsschritt abgetrennt werden müssen.

Insbesondere geeignet ist Wasserstoffperoxid in Form einer wässrigen Lösung mit einem Gehalt an Wasserstoffperoxid von mehr als 10 Gew.-%, vorzugsweise von 30 bis 50 Gew.-%. Wasserstoffperoxidlösungen mit einem geringeren Wirkungsgehalt sind wegen des Eintrags einer zu hohen Menge an Wasser, das anschließend wieder entfernt werden muss, nicht zu empfehlen. Bei zu hohen Wasserstoffperoxidkonzentrationen sind aufwendige und kostspielige Sicherheitsvorkehrungen bei der Handhabung zu beachten.

Man setzt dem zu behandelnden Rohester die peroxidische Verbindung in einer solchen Menge zu, dass ihr Wirkgehalt in dem gesamten Gemisch von 0,03 bis 0,3 Gew.-%, vorzugsweise von 0,08 bis 0,3 Gew.-%, beträgt. Bei zu geringen Wirkkonzentrationen reicht die Entfärbekraft nicht mehr aus, um hellfarbige Polyolester mit ausreichender Qualität zu erhalten. Bei zu hohen Wirkkonzentrationen ist mit unkontrollierten Abbaureaktionen der Esterverbindungen zu rechnen.

Die Behandlung mit peroxidischen Verbindungen erfolgt im Allgemeinen bei erhöhter Temperatur, vorzugsweise bei Temperaturen von 70 bis 160°C, vorzugsweise 100 bis 120°C, wobei auch niedrige Temperaturen, beispielsweise Raumtemperatur oder darunter, nicht ausgeschlossen werden. Die Behandlungsdauer kann über einen weiten Bereich gewählt werden. Sie sollte nicht zu kurz aber auch nicht zu lang sein und kann durch einfache Vorversuche ermittelt werden. Im Allgemeinen beträgt die Behandlungsdauer 0,5 bis 4 Stunden. Bei kürzeren Behandlungsdauern wird kein positiver Einfluss auf die Farbzahl beobachtet, bei zu langen Behandlungszeiten ist auf Grund des anwesenden Wassers und des Oxidationsmittels eine verstärkte Esterspaltung und ein unkontrollierter Abbau des Polyolester-Gerüstes zu befürchten. Ebenfalls wird bei zu langen Behandlungszeiten unnötig Reaktorvolumen belegt.

Die jeweiligen Bedingungen der Behandlung mit der peroxidischen Verbindung sind auf den jeweiligen Polyolester zuzuschneiden, um eine optimale Entfärbung auf der einen Seite zu erzielen aber auf der anderen Seite Abbaureaktionen des Polyolesters möglichst zu vermeiden. Insbesondere bei Polyolestern auf Basis von Etherdiolen, wie zum Beispiel Triethylenglykol oder Tetraethylenglykol, kann ein verstärkter Abbau des Ethergerüstes einsetzen, wenn die Bedingungen bei der Behandlung mit der peroxidischen Verbindung, wie Temperatur, Einwirkungsdauer und Konzentration nicht gezielt auf den jeweiligen Polyolester eingestellt werden.

Nach der oxidativen Behandlung wird der Rohester ohne weitere Zwischenschritte unmittelbar anschließend einer Behandlung mit Wasserdampf unterzogen, die beispielsweise in einfacher Form durch Einleiten von Wasserdampf in das Rohprodukt erfolgen kann. Ein Vorteil der Wasserdampfbehandlung ist, dass in ihrem Verlauf überschüssige peroxidische Verbindungen zerstört werden und Reste der Ausgangsverbindungen mit dem Wasserdampf entfernt werden. Auch größere Mengen noch vorhandenen Wassers werden durch die Wasserdampfbehandlung ausgetrieben. Gleichzeitig wird durch diese Maßnahme die Farbzahl und die Farbstabilität des Rohesters verbessert.

Die Wasserdampfbehandlung wird im Allgemeinen bei Normaldruck durchgeführt, obwohl die Anwendung eines leichten Unterdrucks zweckmäßigerweise bis 400 hPa nicht ausgeschlossen ist. Die Wasserdampfbehandlung wird bei Temperaturen von 170 bis 220°C und insbesondere von 170 bis 200°C, durchgeführt und richtet sich auch nach den physikalischen Eigenschaften der jeweils herzustellenden Polyolester.

Bei dem Prozessschritt der Wasserdampfbehandlung erweist es sich als zweckmäßig, während der Aufheizperiode bis zum Erreichen der Arbeitstemperatur möglichst schonend vorzugehen, um das Gemisch aus Rohester und zugesetzter peroxidischer Verbindung auf die erforderliche Temperatur für die Wasserdampfbehandlung zu erhitzen.

Die Dauer der Wasserdampfbehandlung lässt sich durch Routineversuche ermitteln und sie wird über einen Zeitraum von 0,5 bis 5 Stunden durchgeführt. Eine zu lange Wasserdampfbehandlung führt zu einer ungewünschten Erhöhung der Farbzahl des Polyolesters und ist daher zu vermeiden. Auch beobachtet man eine verstärkte Abbaureaktion des Polyolesters zu sauer reagierenden Verbindungen, deren Gehalt sich in einem Anstieg der Neutralisationszahl oder Säurezahl beispielsweise bestimmt nach DIN EN ISO 3682/ASTM D 1613 zeigt. Bei einer zu kurzen Behandlungsdauer ist die Zerstörung der überschüssigen peroxidischen Verbindung und gebildeter Spuren von organischen Peroxiden nicht vollständig und der gewünschte Polyolester weist noch eine zu hohe unerwünschte Peroxidzahl, ausgedrückt in Milliäquivalent Sauerstoff pro Kilogramm Produkt und bestimmt nach ASTM E 298, auf. Auch wird bei zu kurzer Behandlungsdauer nur ein geringer vorteilhafter Effekt auf die Farbzahl des Polyolesters beobachtet.

Wie bei der Behandlung mit der peroxidischen Verbindung sind auch bei der sich unmittelbar anschließenden Wasserdampfbehandlung die Bedingungen, wie Temperatur, Druck und Dauer gezielt auf den jeweiligen Polyolester einzustellen, um ein optimales Ergebnis in Bezug auf die Farbzahl des Polyolesters zu erzielen und um Restgehalte an Ausgangsverbindungen, Wasser sowie an Peroxidspuren möglichst zu minimieren und gleichzeitig Abbaureaktionen zu unterdrücken. Insbesondere bei Polyolestern auf Basis von Etherdiolen, wie zum Beispiel Triethylenglykol oder Tetraethylenglykol, sind die Bedingungen bei der Wasserdampfbehandlung auf den jeweiligen Polyolester genau zuzuschneiden, um den unerwünschten Abbau der Etherkette zu unterbinden.

Bemerkenswerterweise weist das vom gewünschten Polyolester abgetrennte Wasserdampfdestillat, das nach Kondensation des aus dem Reaktionsteil abgeführten Wasserdampfes anfällt, eine vergleichsweise hohe Peroxidzahl auf. Im technischen Betrieb kann der Anfall großer Wasserdampf- und destillatmengen mit einer hohen Peroxidzahl sich aus sicherheitstechnischen Gründen als problematisch erweisen, da sich in den aufgesetzten Kolonnen und Destillatvorlagen organische und gegebenenfalls anorganischen Peroxide aufkonzentrieren können. Es hat sich als zweckmäßig erwiesen, den abgeführten und mit Wasser sowie unumgesetzten Ausgangsverbindungen beladenen Wasserdampf, in dem auch Peroxide enthalten sind, mit Edelmetallen der Gruppen 9 bis 11 des Periodensystems der Elemente (gemäß IUPAC-Empfehlung 1985), beispielsweise mit Palladium oder Platin, in Kontakt zu bringen. Mit dieser Maßnahme können die im Wasserdampf vorhandenen Peroxidverbindungen zerstört werden. Das Inkontaktbringen erfolgt gasförmig bei der Temperatur des abgeführten Wasserdampfes in Gegenwart der Edelmetalle, indem beispielsweise der Wasserdampf über einen festangeordneten handelsüblichen Edelmetallkatalysator, der entweder geträgert oder ungeträgert sein kann, geleitet wird. Beispielsweise können in einem Kolonnenteil, der auf dem Reaktorteil angebracht ist, feste Einbauten installiert werden, die eine gewebeartige oder porenartige Struktur, beispielsweise eine rechteckige, wabenförmige, runde oder sonstige übliche Struktur, aufweisen, auf denen die Edelmetalle aufgebracht sind und durch deren Kanälen der durch den Rohester geleitete und nunmehr abgeführte, gasförmige und beladene Wasserdampf vorbeistreicht. Ist das Edelmetall auf einem Träger aufgebracht, eignen sich die für Edelmetallkatalysatoren in der Technik gebräuchlichen Träger wie Siliziumdioxid, Aluminiumoxid, Aktivkohle, Titandioxid oder Zirkoniumdioxid in ihren unterschiedlichen Erscheinungsformen.

Auch feste Anordnungen aus Edelmetallen, beispielsweise Gewebe, Netze, Geflechte, Drähte, Knäuele oder Schwämme können in dem Kolonnenteil vorgesehen werden, um mit dem Wasserdampf ausgetriebene Peroxidverbindungen zu zerstören.

Auch das abgetrennte, kondensierte flüssige Destillat, in dem Peroxide angereichert sein können, kann mit Edelmetallen der Gruppen 9 bis 11 des Periodensystems der Elemente zur Zerstörung noch vorhandener Peroxidverbindungen behandelt werden, beispielsweise bei Eigentemperatur mit handelsüblichen geträgerten oder ungeträgerten Edelmetallkatalysatoren, die fest angeordnet oder in Suspension eingesetzt werden können. Auch kann eine übliche feste Anordnung aus Edelmetallen, beispielsweise ein Gewebe, ein Geflecht oder Drähte, beispielsweise ein Platinnetz, mit dem abgetrennten, flüssigen Destillat in Kontakt gebracht werden.

An die Wasserdampfbehandlung schließt sich die Trocknung des Polyolesters an, beispielsweise in dem man ein inertes Gas durch das Produkt bei erhöhter Temperatur leitet. Es kann auch bei erhöhter Temperatur gleichzeitig ein Unterdruck angelegt werden und gegebenenfalls ein inertes Gas durch das Produkt geleitet werden. Auch ohne Einwirken eines inerten Gases kann nur bei erhöhter Temperatur oder nur bei geringerem Druck gearbeitet werden. Die jeweiligen Trocknungsbedingungen, wie Temperatur, Druck und Dauer lassen sich durch einfache Vorversuche ermitteln und sind auf den jeweiligen Polyolester zuzuschneiden. Im Allgemeinen arbeitet man bei Temperaturen im Bereich von 80 bis 250°C, vorzugsweise 100 bis 180°C und bei Drücken von 0,2 bis 500 hPa, vorzugsweise 1 bis 200 hPa, und insbesondere 1 bis 20 hPa. Nach beendeter Trocknung wird ein hellfarbiger Polyolester als Rückstand erhalten, ohne dass ein Filtrationsschritt erforderlich ist, um spezifikationsgerechtes Produkt zu gewinnen. In wenigen Ausnahmefällen kann ein Filtrationsschritt nach der Wasserdampfbehandlung oder nach der Trocknung erforderlich werden, wenn beispielsweise feste Katalysatorreste nach Beendigung der Veresterungsreaktion und nach Abtrennung unumgesetzter Ausgangsverbindungen und damit vor der Aufarbeitung des Reaktionsgemisches nicht vollständig entfernt werden.

In einer besonderen Ausgestaltung des erfindungsgemäßen Verfahrens schließt sich die Trocknung des zurückgebliebenen Polyolesters unmittelbar an die Wasserdampfbehandlung ohne weitere Zwischenschritte an.

Die Reaktion von Polyolen und aliphatischen Monocarbonsäuren kann ohne Einsatz eines Katalysators durchgeführt werden. Diese Variante der Umsetzung hat den Vorteil, dass man vermeidet, dem Reaktionsgemisch Fremdstoffe zuzuführen, die zu einer unerwünschten Verunreinigung des Polyolesters führen können. Allerdings muss man dann im Allgemeinen höhere Reaktionstemperaturen einhalten, weil nur so gewährleistet ist, dass die Umsetzung mit ausreichender, d. h. wirtschaftlich vertretbarer Geschwindigkeit abläuft. Zu beachten ist in diesem Zusammenhang, dass die Steigerung der Temperatur zu einer thermischen Schädigung des Polyolesters führen kann. Daher lässt sich nicht immer die Verwendung eines Katalysators, der die Umsetzung erleichtert und die Reaktionsgeschwindigkeit erhöht, vermeiden. Häufig kann der Katalysator ein Überschuss der aliphatischen Monocarbonsäure sein, die gleichzeitig Reaktionskomponente des Polyols ist, so dass die Reaktion autokatalytisch abläuft. Im Übrigen sind die üblichen Veresterungskatalysatoren zur Beeinflussung der Reaktionsgeschwindigkeit geeignet, wie Schwefelsäure, Ameisensäure, Polyphosphorsäure, Methansulfonsäure oder p-Toluolsulfonsäure und ebenso Kombinationen derartiger Säuren. Ebenfalls verwendet werden können metallhaltige Katalysatoren, wie Titan, Zirkonium oder Zinn enthaltende Katalysatoren, beispielsweise die entsprechenden Alkoholate oder Carboxylate. Auch unter Reaktionsbedingungen feste, im Reaktionssystem unlösliche, katalytisch wirksame Verbindungen wie Alkali- oder Erdalkalihydrogensulfate, beispielsweise Natriumhydrogensulfat können eingesetzt werden, obwohl der Einsatz fester Katalysatoren auf wenige Ausnahmefälle beschränkt ist, denn feste Katalysatoren müssen nach Beendigung der Veresterung aus dem Reaktionsgemisch abfiltriert werden. Gegebenenfalls ist auch noch während der Aufarbeitung des rohen Polyolesters eine zusätzliche Feinfiltration erforderlich um letzte Reste des festen Katalysators zu entfernen. Die Menge des eingesetzten Katalysators kann sich über einen weiten Bereich erstrecken. Es können sowohl 0,001 Gew.-% als auch 5 Gew.-% Katalysator, bezogen auf das Reaktionsgemisch, verwendet werden. Da größere Katalysatormengen jedoch kaum Vorteile ergeben, beträgt die Katalysatorkonzentration üblicherweise 0,001 bis 1,0, vorzugsweise 0,01 bis 0,5 Gew.-%, jeweils bezogen auf das Reaktionsgemisch. Zweckmäßig entscheidet man gegebenenfalls durch Vorversuche für jeden Einzelfall, ob ohne Katalysator bei höherer Temperatur oder mit Katalysator bei niedrigerer Temperatur zu arbeiten ist.

Bei der Veresterung lässt man das Polyol mit überschüssiger Monocarbonsäure reagieren und vorzugsweise ohne Zugabe eines Katalysators, so dass die überschüssige Monocarbonsäure selbst als Katalysator wirkt. Auch lässt sich überschüssige Monocarbonsäure, die einen niedrigeren Siedepunkt als das eingesetzte Polyol aufzuweisen hat, auf einfache Weise destillativ aus dem Rohester abtrennen und ein Filtrationsschritt ist aufgrund der Vermeidung fester Katalysatoren entbehrlich. Die aliphatische Monocarbonsäure wird in einem 10 bis 50%-igen molaren, vorzugsweise in einem 20 bis 40%-igen molaren Überschuss je Mol zu veresternder Hydroxylgruppe des Polyols eingesetzt.

Das gebildete Reaktionswasser wird im Laufe der Reaktion zusammen mit der überschüssigen Monocarbonsäure aus dem Reaktionsgefäß abdestilliert und in einen nachgeschalteten Phasentrenner geleitet, in dem sich die Monocarbonsäure und Wasser je nach ihren Löslichkeitseigenschaften auftrennen. Gegebenenfalls bildet die eingesetzte Monocarbonsäure mit Wasser unter den Reaktionsbedingungen auch ein Azeotrop und vermag als Schleppmittel das Reaktionswasser zu entfernen. Aus dem Wasseranfall kann der Reaktionsverlauf verfolgt werden. Das abgeschiedene Wasser wird aus dem Prozess entfernt während die Monocarbonsäure aus dem Phasentrenner wieder in das Reaktionsgefäß zurückfließt. Die Zugabe eines weiteren organischen Lösungsmittels, wie Hexan, 1-Hexen, Cyclohexan, Toluol, Xylol oder Xylolisomerengemische, das die Aufgabe des Azeotropbildners übernimmt, ist nicht ausgeschlossen, jedoch auf wenige Ausnahmefälle beschränkt. Der Azeotropbildner kann bereits zu Beginn der Veresterungsreaktion oder nach Erreichen höherer Temperaturen zugesetzt werden. Wenn die theoretisch zu erwartende Wassermenge angefallen ist oder die Hydroxylzahl, beispielsweise bestimmt nach DIN 53240, unter einen festgelegten Wert gefallen ist, beendet man die Reaktion indem man den Reaktionsansatz abkühlen lässt.

Die Reaktion zwischen Polyol und aliphatischer Monocarbonsäure setzt in Abhängigkeit von den Einsatzmaterialien im Bereich von etwa 120 bis 180°C ein und kann auf unterschiedlich ausgestaltete Weise zu Ende geführt werden.

Nach einer Ausgestaltung des erfindungsgemäßen Verfahrens wird zunächst ausgehend von Raumtemperatur auf eine Temperatur bis auf maximal 280°C, vorzugsweise bis auf 250°C erhitzt und bei konstant gehaltener Temperatur der Druck ausgehend von Normaldruck stufenweise erniedrigt, um die Entfernung des Reaktionswassers zu erleichtern. Die Wahl der Druckstufen, ob ein-, zwei- oder mehrstufig, sowie der auf der jeweiligen Stufe einzustellende Druck kann über einen weiten Bereich variiert und den jeweiligen Bedingungen angepasst werden. Beispielsweise kann in einer ersten Stufe der Druck ausgehend von Normaldruck zunächst bis auf 600 hPa erniedrigt werden und anschließend die Reaktion bei einem Druck von 300 hPa zu Ende geführt werden. Bei diesen Druckangaben handelt es sich um Richtwerte, die zweckmäßig eingehalten werden.

Neben der Variation des Drucks kann ebenfalls auch die Temperatur ausgehend von Raumtemperatur während der Veresterungsreaktion ein-, zwei- oder mehrstufig verändert werden, so dass bei konstant eingestelltem Druck die Temperatur von Stufe zu Stufe erhöht wird, üblicherweise bis auf eine maximale Temperatur von 280°C. Es hat sich aber als zweckmäßig erwiesen, bei von Stufe zu Stufe ansteigender Temperatur auf maximal 280°C zu erhitzen und auch den Druck von Stufe zu Stufe zu erniedrigen. Beispielsweise kann die Veresterungsreaktion ausgehend von Raumtemperatur in einer ersten Stufe bei einer Temperatur bis auf 190°C geführt werden. Ebenfalls wird ein verminderter Druck bis auf 600 hPa angelegt, um das Austreiben des Reaktionswassers zu beschleunigen. Nach Erreichen der Temperaturstufe von 190°C wird der Druck nochmals bis auf 300 hPa erniedrigt und die Veresterungsreaktion bei einer Temperatur bis auf 250°C zu Ende geführt. Bei diesen Temperatur- und Druckangaben handelt es sich um Richtwerte, die zweckmäßiger Weise eingehalten werden. Die einzustellenden Temperatur- und Druckbedingungen auf den jeweiligen Stufen, die Zahl der Stufen sowie die jeweilige Temperaturerhöungs- oder Druckminderungsrate pro Zeiteinheit können über einen weiten Bereich variiert werden und entsprechend den physikalischen Eigenschaften der Ausgangsverbindungen und der Reaktionsprodukte angepasst werden, wobei die Temperatur- und Druckbedingungen der ersten Stufe ausgehend von Normaldruck und Raumtemperatur eingestellt werden. Besonders zweckmäßig hat es sich erwiesen, die Temperatur in zwei Stufen zu erhöhen und den Druck in zwei Stufen zu erniedrigen.

Die untere Grenze des einzustellenden Drucks hängt von den physikalischen Eigenschaften, wie Siedepunkte und Dampfdrücke, der Ausgangsverbindungen sowie der gebildeten Reaktionsprodukte ab und wird auch durch die Anlagenapparate festgelegt. Ausgehend von Normaldruck kann innerhalb dieser Grenzwerte stufenweise mit von Stufe zu Stufe abnehmenden Drücken gearbeitet werden. Die obere Temperaturgrenze, üblicherweise 280°C, ist einzuhalten, um die Bildung von Zersetzungsprodukten, die u. a. farbschädigend wirken, zu vermeiden. Die untere Grenze der Temperaturstufen wird durch die Reaktionsgeschwindigkeit bestimmt, die noch ausreichend hoch sein muss, um die Veresterungsreaktion innerhalb einer vertretbaren Zeit abzuschließen. Innerhalb dieser Grenzwerte kann stufenweise mit von Stufe zu Stufe ansteigenden Temperaturen gearbeitet werden.

Das nach Beendigung der Umsetzung anfallende Reaktionsgemisch enthält neben dem Polyolester als erwünschtem Reaktionsprodukt gegebenenfalls nicht umgesetzte Ausgangsstoffe, insbesondere noch überschüssige aliphatische Monocarbonsäure, da nach dem erfindungsgemäßen Verfahren mit einem Säureüberschuss gearbeitet wird. Zur Aufarbeitung destilliert man überschüssige und nichtumgesetzte Ausgangsstoffe ab, zweckmäßigerweise unter Anlegen eines verminderten Drucks. Um saure Katalysatoren, wie gelöste Schwefelsäure oder festes Kaliumhydrogensulfat, falls in der Veresterungsstufe zugesetzt, und um letzte Reste acider Bestandteile zu entfernen, kann auch noch eine Behandlung mit einem alkalischen Reagenz, z. B. mit einer wässrigen Soda- oder Natriumhydroxidlösung oder, in Ausnahmefällen, eine Filtration, vorgesehen werden.

Im Anschluss wird der von den unumgesetzten Ausgangsverbindungen und gegebenenfalls anwesenden Katalysator befreite Rohester nach der erfindungsgemäßen Maßnahme umfassend die Behandlung mit peroxidischen Verbindungen, die unmittelbar anschließende Wasserdampfbehandlung und die abschließende Trocknung aufgearbeitet, wobei auf die Verwendung von üblichen Adsorbentien, wie Aktivkohle, oberflächenreiche Polykieselsäuren, wie Silicagele (Kieselgel-Xerogele), Kieselgel, Kieselguhr, oberflächenreiche Aluminiumoxide und Aluminiumoxidhydrate, mineralische Materialien, wie Tone oder Carbonate, während der Aufarbeitung verzichtet wird. Ohne den Einsatz von Adsorbentien werden hellfarbige Polyolester mit einer ausreichend geringen Peroxidzahl erhalten, die auch den übrigen Spezifikationen, wie Wassergehalt, Restsäuregehalt und Restgehalt an Monoester genügen. Der gereinigte Polyolester verbleibt während der Trocknung als Rückstand im Reaktionsgefäß mit hervorragender Qualität und ein zusätzlicher Filtrationsschritt ist im Allgemeinen nicht erforderlich und nur auf wenige Ausnahmefälle beschränkt.

Die als Ausgangsstoffe für das erfindungsgemäße Verfahren eingesetzten mehrwertigen Alkohole oder Polyole genügen der allgemeinen Formel

H-(-O-[-CR¹R²-]ₘ-)ₒ-OH

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.

Als Polyole, die nach dem erfindungsgemäßen Verfahren zu hellfarbigen Polyolestern umgesetzt werden können, eignen sich beispielsweise Di-Trimethylolpropan, Di-Pentaerythrit.

Als weitere Polyole kommen die Oligomeren von Ethylenglykol und 1,2-Propylenglykol, insbesondere die Etherdiole Di-, Tri- und Tetraethylenglykol oder Dipropylenglykol, Tripropylenglykol oder Tetrapropylenglykol in Betracht. Ethylen- und Propylenglykole sind industriell produzierte Chemikalien. Basissubstanz zu ihrer Herstellung ist Ethylenoxid und Propylenoxid, aus dem man 1,2-Ethylenglykol und 1,2-Propylenglykol durch Erhitzen mit Wasser unter Druck gewinnt. Diethylenglykol erhält man durch Ethoxylierung aus Ethylenglykol. Triethylenglykol fällt, wie Tetraethylenglykol, als Nebenprodukt bei der Hydrolyse von Ethylenoxid zur Herstellung von Ethylenglykol an. Beide Verbindungen können auch durch Umsetzung von Ethylenglykol mit Ethylenoxid synthetisiert werden. Dipropylenglykol, Tripropylenglykol, Tetrapropylenglykol und höhere Propoxylierungsprodukte sind aus der mehrfachen Addition von Propylenoxid an 1,2-Propylenglykol zugänglich.

Zur Gewinnung von hellfarbigen Polyolestern nach dem erfindungsgemäßen Prozess setzt man lineare oder verzweigte, aliphatische Monocarbonsäuren mit 3 bis 20 Kohlenstoffatomen im Molekül ein. Obgleich man gesättigten Säuren in vielen Fällen den Vorzug gibt, können, in Abhängigkeit vom jeweiligen Anwendungsgebiet der Weichmacher oder Schmiermittel, auch ungesättigte Carbonsäuren als Reaktionskomponente zur Estersynthese verwendet werden. Beispiele für Monocarbonsäuren als Bausteine von Polyolestern sind Propionsäure, n-Buttersäure, Isobuttersäure, n-Pentansäure, 2-Methylbuttersäure, 3-Methylbuttersäure, 2-Methylpentansäure,n-Hexansäure, 2-Ethylbuttersäure, n-Heptansäure, 2-Methylhexansäure, Cyclohexancarbonsäure, 2-Ethylhexansäure, n-Nonansäure, 2-Methyloctansäure, Isononansäure, 3,5,5-Trimethylhexansäure, 2-Propylheptansäure, 2-Methylundecansäure, Isoundecancarbonsäure, Tricyclodecancarbonsäure und Isotridecancarbonsäure. Besonders bewährt hat sich das neue Verfahren für die Herstellung von Polyolestern der oligomeren Ethylenglykole sowie der oligomeren Propylenglykole mit C₄- bis C₁₃- bzw. C₅- bis C₁₀-Monocarbonsäuren sowie zur Herstellung von Polyolestern auf Basis von Di-Trimethylolpropan.

Die Polyolester der Oligomeren des Ethylenglykols eignen sich hervorragend als Weichmacher für alle gängigen hochpolymeren thermoplastischen Stoffe. Besonders bewährt haben sie sich als Zusatz zu Polyvinylbutyral, das mit Glykolestern versetzt als Zwischenschicht zur Herstellung von Mehrschichten- oder Verbundgläsern verwendet wird. Sie können ebenfalls als Koaleszenzmittel oder Filmbildehilfsmittel in wässrigen Dispersionen von Kunststoffen, die als Beschichtungsmittel vielfältige Anwendung finden, eingesetzt werden. Nach dem erfindungsgemäßen Herstellungsverfahren lassen sich auf einfache Weise ohne die Verwendung von üblichen Adsorbentien Polyolester mit ausgezeichneten Farbeigenschaften herstellen, die auch weiteren Qualitätsansprüchen, wie geringem Geruch oder einer geringen Säurezahl genügen. Besonders eignet sich das erfindungsgemäße Verfahren zur Herstellung von Triethylenglykol-di-2-ethylhexanoat (3G8 Ester), Tetraethylenglykol-di-n-heptanoat (4G7 Ester), Triethylenglykol-di-2-ethylbutyrat (3G6 Ester), Triethylenglykol-di-n-heptanoat (3G7 Ester) oder Tetraethylenglykol-di-2-ethylhexanoat (4G8 Ester).

Das erfindungsgemäße Verfahren kann kontinuierlich oder absatzweise in den für die chemische Technik typischen Reaktionsapparaten durchgeführt werden. Bewährt haben sich Rührkessel oder Reaktionsrohre, die mit einer Heizvorrichtung und mit einem aufgesetzten Kolonnenteil ausgestattet sind.

In den folgenden Beispielen wird das erfindungsgemäße Verfahren näher erläutert, es ist jedoch nicht auf die beschriebene Ausführungsform beschränkt.

### Ausführungsbeispiele

Für die Versuche zur Farbaufhellung kam rohes Triethylenglykol-di-2-ethylhexanoat mit einer Farbzahl von 131 Hazen-Einheiten zum Einsatz, das durch Veresterung von Triethylenglykol mit einer 2,3 molaren Menge an 2-Ethylhexansäure ohne Katalysator und Schleppmittelzusatz gewonnen wurde. Der gaschromatographisch ermittelte Gehalt (Gew.-%) an Triethylenglykol-di-2-ethylhexanoat betrug 97,7%, an Triethylenglykol-mono-2-ethylhexanoat 1,2% und der Rest auf 100% betrug 1,1 %.
Die Aufarbeitung des rohen Triethylenglykol-di-2-ethylhexanoats wurde mit jeweils 300 g Rohprodukt in einem beheizbaren 1 Liter-Vierhalskolben durchgeführt, der mit Rührer, Innenthermometer und Tropftrichter ausgestattet wurde. Nach Zugabe der wässrigen Wasserstoffperoxidlösung und Rühren gemäß den nachfolgend beschriebenen Reaktionsbedingungen wurde für die sich anschließende Wasserdampfdestillation der Tropftrichter durch eine Destillationsbrücke mit einer 1 Liter-Vorlage ersetzt und der 1 Liter-Vierhalskolben mit einem Tauchrohr zur Durchleitung von Wasserdampf versehen. In der Destillationskolonne wurde ein Platinnetz angebracht, über das der ausgetriebene und mit Peroxiden beladene Wasserdampf geleitet wurde.

Nach Durchführung der Wasserdampfdestillation gemäß den nachfolgend beschriebenen Bedingungen unterbrach man die Zufuhr von Wasserdampf und man legte zur abschließenden Trocknung über die Destillationsbrücke einen Unterdruck an. Man erhielt als Rückstand einen hellfarbigen, spezifikationsgerechten Polyolester ohne den Einsatz von Adsorbentien.

### Beispiel 1:

Die Behandlung des rohen Triethylenglykol-di-2-ethylhexanoats mit einer wässrigen Wasserstoffperoxidlösung erfolgte unter folgenden Bedingungen:

| | |
|---|---|
| Konzentration der wässrigen H₂O₂-Lösung | 30 Gew.-% |
| Mengen H₂O₂, absolut bezogen auf das gesamte Reaktionsgemisch | 0,10 Gew.-% |
| Reaktionstemperatur | 120°C |
| Reaktionsdauer | 1 Stunde |

Die unmittelbar anschließende Wasserdampfdestillation wurde unter Verwendung eines Platinnetzes unter folgenden Bedingungen durchgeführt:

| | |
|---|---|
| Arbeitstemperatur der Wasserdampfdestillation | 180°C |
| Behandlungsdauer | 1 Stunde |

Anschließend wurden folgende Trocknungsbedingungen eingestellt:

| | |
|---|---|
| Druck | 10 hPa |
| Trocknungstemperatur | 140°C |
| Trocknungsdauer | 0,5 h |

Nach Abschluss der Aufarbeitung erhielt man einen hellfarbigen Polyolester mit folgenden gaschromatographisch ermittelten Gehalten:

| | |
|---|---|
| Gehalt an Triethylenglykol-di-2-ethylhexanoat | 97,9 Gew.-% |
| Gehalt an Triethylenglykol-mono-2-ethylhexanoat | 0,9 Gew.-% |
| Rest | 1,2 Gew.-% |

und folgenden Kennzahlen:

| | |
|---|---|
| Farbzahl nach Hazen (DIN ISO 6271) | 29 |
| Neutralisationszahl (mg KOH/g, DIN EN ISO 3682/ ASTM D 1613) | 0,05 |
| Wassergehalt (Gew.-%, DIN 51777 Teil 1) | 0,05 |
| Peroxidgehalt (mäq O/kg, ASTM E 298) | 1,15 |

Im Destillat der Wasserdampfdestillation wurde ein Peroxidgehalt von 3,0 mäq O/kg gefunden.

### Beispiel 2:

Beispiel 2 wurde gemäß dem Beispiel 1 durchgeführt mit der einzigen Ausnahme, dass die Wasserdampfdestillation ohne die Verwendung eines Platinnetzes erfolgte. Das erhaltene Destillat wies einen Peroxidgehalt von 13 mäq O/kg auf. Die Kennzahlen des gereinigten Polyolesters entsprachen den nach Beispiel 1 ausgewiesenen Werten.

### Beispiel 3 (Vergleichsbeispiel):

Herstellung von NPG-di-2-ethylhexanoat mit nachfolgender Behandlung mit Wasserstoffperoxid

Die Veresterung von Neopentylglykol mit 2-Ethylhexansäure wurde in einem beheizbaren 1 Liter - Vierhalskolben durchgeführt, der mit Rührer, Innenthermometer und einem Wasserabscheider ausgestattet wurde.

In dem Kolben wurden 312,75 Gramm (3,00 mol) Neopentylglykol und 966,89 Gramm (6,70 mol) 2-Ethylhexansäure vorgelegt. Unter Rühren und unter Anlegen eines Unterdrucks von 600 hPa wurde der Ansatz auf 200°C erhitzt und man ließ über 2 Stunden unter diesen Bedingungen reagieren. Anschließend wurde der Druck schrittweise auf 500 hPa reduziert und gebildetes Reaktionswasser an dem Wasserabscheider abgenommen. Der Reaktionsverlauf wurde durch kontinuierliche Auswaage des über den Wasserabscheider ausgeschleusten Wassers sowie durch den Verlauf der Hydroxylzahl verfolgt. Nach insgesamt 8 Stunden Reaktionszeit wurde bei einer Resthydroxylzahl von 4,2 mg KOH/g (nach DIN 53240) die Reaktion beendet.

Anschließend wurde über einen Zeitraum von 2 Stunden bei einer Temperatur von 190°C und bei einem Druck von 95 hPa und weiterhin über 30 Minuten bei einer Temperatur von 130°C und einem Druck von 6 hPa die überschüssige 2-Ethylhexansäure abdestilliert. Es folgte die Behandlung mit einer 30 Gew.-%igen Wasserstoffperoxidlösung in einer Menge von 0,1 Gew.-% absolut Wasserstoffperoxid, bezogen auf den Reaktionsansatz, bei einer Temperatur von 120°C über einen Zeitraum von 1 Stunde.

Die nachfolgende Wasserdampfdestillation wurde bei Normaldruck unter Durchleiten von Wasserdampf bei einer Temperatur von 180°C über einen Zeitraum von 1 Stunde durchgeführt. Anschließend unterbrach man die Wasserdampfdestillation und legte zur abschließenden Trocknung über die Destillationsbrücke einen Druck von 10 hPa an. Die Trocknung wurde über einen Zeitraum von 30 Minuten bei 140°C durchgeführt. Man erhielt als Rückstand hellfarbiges Neopentylglykol-di-2-ethylhexanoat mit folgenden Kennzahlen

Gaschromatographisch ermittelte Gehalte:

| | |
|---|---|
| Gehalt an Neopentylglykol-di-2-ethylhexanoat | 92,7 Gew.-% |
| Gehalt an Neopentylglykol-mono-2-ethylhexanoat | 6,2 Gew.-% |
| Rest | 1,1 Gew.-% |

### Kennzahlen:

| | |
|---|---|
| Farbzahl nach Hazen (DIN ISO 6271) | 49 |
| Neutralisationszahl (mg KOH/g, DIN EN ISO 3682/ ASTM D 1613) | 0,26 |
| Wassergehalt (Gew.-%, DIN 51777 Teil 1) | 0,02 |
| Peroxidgehalt (mäq O/kg, ASTM E 298) | 1,59 |

### Beispiel 4 (Vergleichsbeispiel):

Die Veresterung von Neopentylglykol mit 2-Ethylhexansäure und die nachfolgende Abtrennung der nicht umgesetzten und überschüssigen 2-Ethylhexansäure wurden gemäß Beispiel 3 durchgeführt. Ohne die Behandlung mit Wasserstoffperoxid erfolgte die anschließende Wasserdampfbehandlung bei 180°C über einen Zeitraum von 30 Minuten und die nachfolgende Trocknung bei 120°C über 15 Minuten. Das im Rückstand angefallene Neopentylglykol-di-2-ethylhexanoat zeigte folgende Kennzahlen:

Gaschromatographisch ermittelte Gehalte:

| | |
|---|---|
| Gehalt an Neopentylglykol-di-2-ethylhexanoat | 92,9 Gew.-% |
| Gehalt an Neopentylglykol-mono-2-ethylhexanoat | 6,2 Gew.-% |
| Rest | 0,9 Gew.-% |

Kennzahlen:

| | |
|---|---|
| Farbzahl nach Hazen (DIN ISO 6271) | 140 |
| Neutralisationszahl (mg KOH/g, DIN EN ISO 3682/ ASTM D 1613) | 0,17 |
| Wassergehalt (Gew.-%, DIN 51777 Teil 1) | 0,02 |

Durch die erfindungsgemäße Maßnahme, das rohe Veresterungsgemisch nach Abtrennung unumgesetzter Ausgangsverbindungen mit Wasserstoffperoxid zu behandeln und anschließend unmittelbar ohne weitere Zwischenschritte eine Wasserdampfbehandlung durchzuführen, lassen sich hellfarbige Polyolester mit hoher Farbstabilität ohne den Einsatz von Adsorbentien produzieren. In einer weiteren Ausgestaltung des erfindungsgemäßen Verfahrens kann der während der Wasserdampfbehandlung ausgetriebene Wasserdampf mit einem Platinnetz in Kontakt gebracht werden. Mit dieser Maßnahme kann der Peroxidgehalt im abgetrennten Destillat deutlich abgereichert werden, wodurch sich sicherheitstechnische Probleme vermeiden lassen, die man bei einem Anfall von Destillatmengen mit einem hohen Peroxidgehalt zu bewältigen hätte.

## Patentansprüche

1. Verfahren zur Farbaufhellung von Polyolestern durch Umsetzung von Polyolen der allgemeinen Formel
H-(-O-[-CR¹R²-]ₘ-)ₒ-OH
in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten, mit linearen oder verzweigten aliphatischen Monocarbonsäuren mit 3 bis 20 Kohlenstoffatomen im Überschuss und anschließende Aufarbeitung des Reaktionsgemisches ohne die Verwendung von Adsorbentien, **dadurch gekennzeichnet, dass** nach Abtrennung unumgesetzter Ausgangsverbindungen das Umsetzungsprodukt mit peroxidischen Verbindungen, ausgewählt aus Wasserstoffperoxid, Alkali- oder Erdalkalimetallperboraten, Alkali- oder Erdalkalimetallpercarbonaten und Alkali- oder Erdalkalimetallperoxophosphaten, mit einem Wirkgehalt von 0,03 bis 0,3 Gew.-%, bezogen auf die gesamte Mischung, behandelt wird, unmittelbar anschließend ohne weitere Zwischenschritte eine Wasserdampfbehandlung bei einer Temperatur von 170 bis 220°C und über einen Zeitraum von 0,5 bis 5 Stunden durchgeführt wird und der zurückgebliebene Polyolester getrocknet wird, mit der Maßgabe, dass die aliphatische Monocarbonsäure einen niedrigeren Siedepunkt als das eingesetzte Polyol aufweist.

2. Verfahren gemäß Anspruch 1, **dadurch gekennzeichnet, dass** der Wirkgehalt an peroxidischer Verbindung 0,08 bis 0,3 Gew.-%, bezogen auf die gesamte Mischung beträgt.

3. Verfahren gemäß Anspruch 1 oder 2, **dadurch gekennzeichnet, dass** man Wasserstoffperoxid in Form einer wässrigen Lösung mit einem Gehalt an Wasserstoffperoxid von mehr als 10 Gew.-%, vorzugsweise von 30 bis 50 Gew.-% verwendet.

4. Verfahren gemäß einem oder mehreren der Ansprüche 1 bis 3, **dadurch gekennzeichnet, dass** die Behandlung mit peroxidischen Verbindungen bei Temperaturen von 70 bis 160°C, vorzugsweise 100 bis 120°C erfolgt.

5. Verfahren gemäß einem oder mehreren der Ansprüche 1 bis 4, **dadurch gekennzeichnet, dass** man die Wasserdampfbehandlung bei einer Temperatur von 170 bis 200°C durchführt.

6. Verfahren gemäß einem oder mehreren der Ansprüche 1 bis 5, **dadurch gekennzeichnet, dass** der bei der Wasserdampfbehandlung abgeführte Wasserdampf gasförmig mit Edelmetallen der Gruppen 9 bis 11 des Periodensystems der Elemente in Kontakt gebracht wird.

7. Verfahren gemäß einem oder mehreren der Ansprüche 1 bis 5, **dadurch gekennzeichnet, dass** der bei der Wasserdampfbehandlung abgeführte Wasserdampf zunächst kondensiert wird und das kondensierte flüssige Destillat mit Edelmetallen der Gruppen 9 bis 11 des Periodensystems der Elemente in Kontakt gebracht wird.

8. Verfahren gemäß Anspruch 6 oder 7, **dadurch gekennzeichnet, dass** die Edelmetalle der Gruppen 9 bis 11 des Periodensystems der Elemente fest angeordnet sind.

9. Verfahren gemäß Anspruch 8, **dadurch gekennzeichnet, dass** die Edelmetalle der Gruppen 9 bis 11 des Periodensystems der Elemente auf einem Träger aufgebracht sind.

10. Verfahren gemäß Anspruch 9, **dadurch gekennzeichnet, dass** als Träger Siliziumdioxid, Aluminiumoxid, Aktivkohle, Titandioxid oder Zirkondioxid verwendet werden.

11. Verfahren gemäß Anspruch 8, **dadurch gekennzeichnet, dass** die Edelmetalle der Gruppen 9 bis 11 des Periodensystems der Elemente in Form eines Gewebes, Netzes, Geflechtes, Drahtes, Knäuels oder Schwammes angeordnet sind.

12. Verfahren gemäß einem oder mehreren der Ansprüche 6 bis 11, **dadurch gekennzeichnet, dass** als Edelmetalle der Gruppen 9 bis 11 des Periodensystems der Elemente Palladium oder Platin verwendet werden.

13. Verfahren gemäß einem oder mehreren der Ansprüche 1 bis 12, **dadurch gekennzeichnet, dass** der Polyolester bei Temperaturen von 80 bis 250°C, vorzugsweise 100 bis 180°C und bei Drücken von 0,2 bis 500 hPa, vorzugsweise 1 bis 200 hPa und insbesondere von 1 bis 20 hPa getrocknet wird.

14. Verfahren gemäß einem oder mehreren der Ansprüche 1 bis 13, **dadurch gekennzeichnet, dass** der zurückgebliebene Polyolester unmittelbar anschließend an die Wasserdampfbehandlung ohne weitere Zwischenschritte getrocknet wird.

15. Verfahren gemäß einem oder mehreren der Ansprüche 1 bis 14, **dadurch gekennzeichnet, dass** man als Polyole Di-Trimethylolpropan, Di-Pentaerythrit, Diethylenglykol, Triethylenglykol, Tetraethylenglykol, Dipropylenglykol, Tripropylenglykol oder Tetrapropylenglykol verwendet.

16. Verfahren gemäß einem oder mehreren der Ansprüche 1 bis 15, **dadurch gekennzeichnet, dass** man als aliphatische Monocarbonsäure Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, 2-Ethylhexansäure, n-Nonansäure, 2-Methyloctansäure, Isononansäure, 3,5,5-Trimethylhexansäure oder 2-Propylheptansäure umsetzt.

17. Verfahren gemäß einem oder mehreren der Ansprüche 1 bis 16 zur Herstellung von Triethylenglykol-di-2-ethylhexanoat, Tetraethylenglykol-di-n-heptanoat, Triethylenglykol-di-2-ethylbutyrat, Triethylenglykol-di-n-heptanoat oder Tetraethylenglykol-di-2-ethylhexanoat.

## Claims

1. Process for lightening the colour of polyol esters of the general formula
H-(-O-[-CR¹R²-]ₘ-)ₒ-OH
in which R¹ and R² are each independently hydrogen, an alkyl radical having 1 to 5 carbon atoms, preferably methyl, ethyl or propyl, or a hydroxyalkyl radical having 1 to 5 carbon atoms, preferably the hydroxymethyl radical, m is an integer of 1 to 10, preferably 1 to 8 and especially 1, 2, 3 or 4, o is an integer of 2 to 15, preferably 2 to 8 and especially 2, 3, 4 or 5, by reacting polyols with linear or branched aliphatic monocarboxylic acids having 3 to 20 carbon atoms in excess and then working up the reaction mixture without the use of adsorbents, **characterized in that** removal of unconverted starting compounds is followed by treating the reaction product with peroxidic compounds, selected from hydrogen peroxide, alkali metal or alkaline earth metal perborates, alkali metal or alkaline earth metal percarbonates and alkali metal or alkaline earth metal peroxophosphates, having an active content of 0.03% to 0.3% by weight, based on the overall mixture immediately thereafter performing a steam treatment without further intermediate steps at a temperature of 170 to 220°C and over a period of 0.5 to 5 hours, and drying the remaining polyol ester, with the proviso that the aliphatic monocarboxylic acid has a lower boiling point than the polyol used.

2. Process according to Claim 1, **characterized in that** the active content of peroxidic compound is 0.08 to 0.3% by weight, based on the overall mixture.

3. Process according to Claim 1 or 2, **characterized in that** hydrogen peroxide is used in the form of an aqueous solution having a hydrogen peroxide content of more than 10% by weight, preferably of 30 to 50% by weight.

4. Process according to one or more of Claims 1 to 3, **characterized in that** the treatment with peroxidic compounds is effected at temperatures of 70 to 160°C, preferably 100 to 120°C.

5. Process according to one or more of Claims 1 to 4, **characterized in that** the steam treatment is performed at a temperature of 170 to 200°C.

6. Process according to one or more of Claims 1 to 5, **characterized in that** the steam removed in the steam treatment is contacted in gaseous form with noble metals of groups 9 to 11 of the periodic table of the elements.

7. Process according to one or more of Claims 1 to 5, **characterized in that** the steam removed in the steam treatment is first condensed and the condensed liquid distillate is contacted with noble metals of groups 9 to 11 of the periodic table of the elements.

8. Process according to Claim 6 or 7, **characterized in that** the noble metals of groups 9 to 11 of the periodic table of the elements are arranged in a fixed form.

9. Process according to Claim 8, **characterized in that** the noble metals of groups 9 to 11 of the periodic table of the elements have been applied to a support.

10. Process according to Claim 9, **characterized in that** the support used is silicon dioxide, aluminium oxide, activated carbon, titanium dioxide or zirconium dioxide.

11. Process according to Claim 8, **characterized in that** the noble metals of groups 9 to 11 of the periodic table of the elements are arranged in the form of a fabric, mesh, braid, wire, coil or sponge.

12. Process according to one or more of Claims 6 to 11, **characterized in that** the noble metals of groups 9 to 11 of the periodic table of the elements used are palladium or platinum.

13. Process according to one or more of Claims 1 to 12, **characterized in that** the polyol ester is dried at temperatures of 80 to 250°C, preferably 100 to 180°C, and at pressures of 0.2 to 500 hPa, preferably 1 to 200 hPa and especially of 1 to 20 hPa.

14. Process according to one or more of Claims 1 to 13, **characterized in that** the remaining polyol ester is dried immediately after the steam treatment without further intermediate steps.

15. Process according to one or more of Claims 1 to 14, **characterized in that** the polyols used are ditrimethylolpropane, dipentaerythritol, diethylene glycol, triethylene glycol, tetraethylene glycol, dipropylene glycol, tripropylene glycol or tetrapropylene glycol.

16. Process according to one or more of Claims 1 to 15, **characterized in that** the aliphatic monocarboxylic acid converted is propionic acid, n-butyric acid, isobutyric acid, n-pentanoic acid, 2-methylbutyric acid, 3-methylbutyric acid, 2-methylpentanoic acid, n-hexanoic acid, 2-ethylbutyric acid, n-heptanoic acid, 2-methylhexanoic acid, 2-ethylhexanoic acid, n-nonanoic acid, 2-methyloctanoic acid, isononanoic acid, 3,5,5-trimethylhexanoic acid or 2-propylheptanoic acid.

17. Process according to one or more of Claims 1 to 16 for preparing triethylene glycol di-2-ethylhexanoate, tetraethylene glycol di-n-heptanoate, triethylene glycol di-2-ethylbutyrate, triethylene glycol di-n-heptanoate or tetraethylene glycol di-2-ethylhexanoate.

## Revendications

1. Procédé d'éclaircissement d'esters de polyols par mise en réaction de polyols de formule générale
H-(-O-[-CR¹R²-]ₘ-)ₒ-OH
dans laquelle R¹ et R² signifient indépendamment l'un de l'autre l'hydrogène, un radical alkyle de 1 à 5 atomes de carbone, de préférence méthyle, éthyle ou propyle, ou un radical hydroxyalkyle de 1 à 5 atomes de carbone, de préférence le radical hydroxyméthyle, m signifie un nombre entier de 1 à 10, de préférence de 1 à 8 et notamment 1, 2, 3 ou 4, o signifie un nombre entier de 2 à 15, de préférence de 2 à 8 et notamment 2, 3, 4 ou 5, avec des acides monocarboxyliques aliphatiques linéaires ou ramifiés de 3 à 20 atomes de carbone en excès, puis traitement du mélange réactionnel sans l'utilisation d'adsorbants, **caractérisé en ce qu'**après la séparation des composés de départ non réagis, le produit de la réaction est traité avec des composés peroxydiques, choisis parmi le peroxyde d'hydrogène, les perborates de métaux alcalins ou alcalino-terreux, les percarbonates de métaux alcalins ou alcalino-terreux, et les peroxophosphates de métaux alcalins ou alcalino-terreux, ayant une teneur active de 0,03 à 0,3 % en poids, par rapport au mélange total, puis directement sans étape intermédiaire supplémentaire, un traitement à la vapeur d'eau est réalisé à une température de 170 à 220 °C et pendant une durée de 0,5 à 5 heures, et l'ester de polyol restant est séché, à condition que l'acide monocarboxylique aliphatique présente un point d'ébullition inférieur au polyol utilisé.

2. Procédé selon la revendication 1, **caractérisé en ce que** la teneur active en composé peroxydique est de 0,08 à 0,3 % en poids, par rapport au mélange total.

3. Procédé selon la revendication 1 ou 2, **caractérisé en ce que** le peroxyde d'hydrogène est utilisé sous la forme d'une solution aqueuse ayant une teneur en peroxyde d'hydrogène de plus de 10 % en poids, de préférence de 30 à 50 % en poids.

4. Procédé selon une ou plusieurs des revendications 1 à 3, **caractérisé en ce que** le traitement avec des composés peroxydiques a lieu à des températures de 70 à 160 °C, de préférence de 100 à 120 °C.

5. Procédé selon une ou plusieurs des revendications 1 à 4, **caractérisé en ce que** le traitement à la vapeur d'eau est réalisé à une température de 170 à 200 °C.

6. Procédé selon une ou plusieurs des revendications 1 à 5, **caractérisé en ce que** la vapeur d'eau déchargée lors du traitement à la vapeur d'eau est mise en contact sous forme gazeuse avec des métaux nobles des groupes 9 à 11 du tableau périodique des éléments.

7. Procédé selon une ou plusieurs des revendications 1 à 5, **caractérisé en ce que** la vapeur d'eau déchargée lors du traitement à la vapeur d'eau est tout d'abord condensée, et le distillat liquide condensé est mis en contact avec des métaux nobles des groupes 9 à 11 du tableau périodique des éléments.

8. Procédé selon la revendication 6 ou 7, **caractérisé en ce que** les métaux nobles des groupes 9 à 11 du tableau périodique des éléments sont agencés de manière fixe.

9. Procédé selon la revendication 8, **caractérisé en ce que** les métaux nobles des groupes 9 à 11 du tableau périodique des éléments sont appliqués sur un support.

10. Procédé selon la revendication 9, **caractérisé en ce que** du dioxyde de silicium, de l'oxyde d'aluminium, du charbon actif, du dioxyde de titane ou du dioxyde de zirconium est utilisé en tant que support.

11. Procédé selon la revendication 8, **caractérisé en ce que** les métaux nobles des groupes 9 à 11 du tableau périodique des éléments sont agencés sous la forme d'un tissu, d'un réseau, d'un treillis, d'un fil, d'une pelote ou d'une éponge.

12. Procédé selon une ou plusieurs des revendications 6 à 11, **caractérisé en ce que** du palladium ou du platine est utilisé en tant que métal noble des groupes 9 à 11 du tableau périodique des éléments.

13. Procédé selon une ou plusieurs des revendications 1 à 12, **caractérisé en ce que** l'ester de polyol est séché à des températures de 80 à 250 °C, de préférence de 100 à 180 °C, et à des pressions de 0,2 à 500 hPa, de préférence de 1 à 200 hPa et notamment de 1 à 20 hPa.

14. Procédé selon une ou plusieurs des revendications 1 à 13, **caractérisé en ce que** l'ester de polyol restant est séché directement après le traitement à la vapeur d'eau sans étape intermédiaire supplémentaire.

15. Procédé selon une ou plusieurs des revendications 1 à 14, **caractérisé en ce que** du di-triméthylolpropane, de la di-pentaérythrite, du diéthylène glycol, du triéthylène glycol, du tétraéthylène glycol, du dipropylène glycol, du tripropylène glycol ou du tétrapropylène glycol est utilisé en tant que polyol.

16. Procédé selon une ou plusieurs des revendications 1 à 15, **caractérisé en ce que** de l'acide propionique, de l'acide n-butyrique, de l'acide isobutyrique, de l'acide n-pentanoïque, de l'acide 2-méthylbutyrique, de l'acide 3-méthylbutyrique, de l'acide 2-méthylpentanoïque, de l'acide n-hexanoïque, de l'acide 2-éthylbutyrique, de l'acide n-heptanoïque, de l'acide 2-méthylhexanoïque, de l'acide 2-éthylhexanoïque, de l'acide n-nonanoïque, de l'acide 2-méthyloctanoïque, de l'acide isononanoïque, de l'acide 3,5,5-triméthylhexanoïque ou de l'acide 2-propylheptanoïque est mis en réaction en tant qu'acide monocarboxylique aliphatique.

17. Procédé selon une ou plusieurs des revendications 1 à 16, pour la fabrication de di-2-éthylhexanoate de triéthylène glycol, de di-n-heptanoate de tétraéthylène glycol, de di-2-éthylbutyrate de triéthylène glycol, de di-n-heptanoate de triéthylène glycol ou de di-2-éthylhexanoate de tétraéthylène glycol.
